# FASCICULE DE BREVET EUROPEEN

(11) **EP 3 407 986 B1**
(45) Date de publication et mention de la délivrance du brevet: **23.10.2019**
(21) Numéro de dépôt: 17706282.5
(22) Date de dépôt: 24.01.2017
(51) Int. Cl.: A63B 23/18, A63B 24/00, A63B 71/06, A63B 21/008, A61M 16/08

(54) **DISPOSITIF D'ACCLIMATATION A L'ALTITUDE ET PROCEDE DE FONCTIONNEMENT DE CE DISPOSITIF**
HÖHENLAGENAKKLIMATISIERUNGSVORRICHTUNG UND VERFAHREN ZUM BETRIEB DAVON
ALTITUDE ACCLIMATISATION DEVICE AND METHOD FOR OPERATING SAID DEVICE

(30) Priorité: 25.01.2016 FR 1650554
(43) Date de publication de la demande: 05.12.2018
(73) Titulaire: Université Grenoble Alpes, 38400 Saint Martin d'Hères (FR); Centre Hospitalier Universitaire Grenoble, 38700 La Tronche (FR); INSERM (Institut National de la Santé et de la Recherche Médicale), 75013 Paris (FR); Universite De Chambery Universite Savoie Mont Blanc, 73000 Chambéry (FR)
(72) Inventeur: VERGÈS, Samuel, 38320 Eybens (FR); WUYAM, Bernard, 38130 Echirolles (FR); RUPP, Thomas, 73000 Chambery (FR)
(74) Mandataire: Bronchart, Quentin
(86) Numéro de dépôt international: PCT/FR2017/050138
(87) Numéro de publication internationale: WO 2017/129886

(56) Documents cités:
- WO-A1-2015/104522
- WO-A1-2015/120435

## Description

### DOMAINE DE L'INVENTION

L'invention concerne un dispositif d'acclimatation à l'altitude et un procédé d'utilisation de ce dispositif.

### ARRIERE PLAN TECHNOLOGIQUE DE L'INVENTION

L'altitude implique une moindre disponibilité en oxygène dans l'air respiré et une moindre oxygénation de l'organisme. Cette hypoxie est bien connue pour être la cause d'une diminution des capacités fonctionnelles physique et/ou cognitive et, dans certains cas, du développement de mal de tête, nausées, fatigue.

Dans le document « Positive Expiratory Pressure Improves Oxygénation in Healthy Subjects Exposed to Hypoxia » de H. Nespoulet et al (publiée dans le journal « PLOS one » de December 2013, Vol 8, issue 12), il a été montré que l'ajout d'une pression expiratoire positive à l'aide d'un masque de ventilation à la bouche d'un sujet en condition d'altitude permettait, grâce à l'augmentation artificielle de la pression intrapulmonaire, de limiter les effets de l'altitude en augmentant l'oxygénation sanguine et musculaire. Un tel masque est par exemple décrit dans le document EP0771578.

Bien qu'ayant permis de valider des hypothèses scientifiques initiales, le dispositif expérimental déployé dans le cadre de l'étude rapportée par le document cité n'est pas adapté à un usage courant. Outre sa configuration complexe, il nécessite également un encadrement par des spécialistes afin de s'assurer de son usage conforme, et de son efficacité.

Il est connu par ailleurs, des documents de brevets référencés WO 2015/120435 A1 et WO 2015/104522 A1, des dispositifs d'acclimatation à l'altitude comprenant chacun un masque de respiration définissant un espace confiné d'air lorsqu'il est placé sur le visage d'un utilisateur, le masque comportant :
- Une valve d'inspiration pour laisser entrer de l'air environnant à l'intérieur de l'espace confiné lors d'une inspiration ;
- Une valve d'expiration pour laisser sortir de l'air avec une résistance déterminée, de sorte à créer une surpression d'air dans l'espace confiné, lors d'une expiration ;
- Un capteur pour établir une information de pression de l'espace confiné d'air,
chaque dispositif d'acclimatation comportant de plus un organe de traitement de l'information de pression configuré pour établir des caractéristiques respiratoires de l'utilisateur et les comparer, respectivement, à au moins une valeur cible, en vue de lui préconiser un ajustement de son mode respiratoire.

### OBJET DE L'INVENTION

Un but de l'invention est de proposer un dispositif d'acclimatation à l'altitude qui soit compact et facilement utilisable sur la base de consignes simples, permettant d'augmenter efficacement l'apport en oxygène à l'organisme de son utilisateur.

### BREVE DESCRIPTION DE L'INVENTION

En vue de la réalisation de ce but, l'objet de l'invention propose un dispositif d'acclimatation à l'altitude comprenant un masque de respiration définissant un espace confiné d'air lorsqu'il est placé sur le visage d'un utilisateur, le masque comportant :
- Une valve d'inspiration pour laisser entrer de l'air environnant à l'intérieur de l'espace confiné lors d'une inspiration ;
- Une valve d'expiration pour laisser sortir de l'air avec une résistance déterminée, de sorte à créer une surpression d'air dans l'espace confiné, lors d'une expiration ;
- Un capteur pour établir une information de pression de l'espace confiné d'air.

Le dispositif d'acclimatation comporte de plus un organe de traitement de l'information de pression configuré pour établir des caractéristiques respiratoires de l'utilisateur et les comparer, respectivement, à au moins une valeur cible, en vue de lui préconiser un ajustement de son mode respiratoire, le dispositif étant essentiellement tel que les caractéristiques respiratoires comprennent la pression maximale dans l'espace confiné lors d'une expiration de valeur cible comprise entre 8 et 12 cmH2O, la durée d'une expiration de valeur cible comprise entre 3,5 et 4,5 secondes, et la durée d'une inspiration de valeur cible comprise entre 1,5 et 2,5 secondes.

Ainsi l'utilisateur du dispositif reçoit les consignes lui permettant d'utiliser de manière conforme le masque respiratoire pour augmenter efficacement l'apport en oxygène à l'organisme de son utilisateur.

Selon d'autres caractéristiques avantageuses et non limitatives de l'invention, prises seules ou en combinaison :
- Le dispositif d'acclimatation comprend également un oxymètre en liaison avec l'organe de traitement.
- Le dispositif d'acclimatation comprend également un organe de préconisation en liaison avec le dispositif de traitement.
- l'organe de traitement est disposé dans ou sur le masque.
- l'organe de traitement est disposé ou est constitué par un dispositif distinct du masque.

L'invention a pour second objet un procédé de fonctionnement d'un dispositif d'acclimatation à l'altitude comprenant un masque de respiration définissant un espace confiné d'air lorsqu'il est placé sur le visage d'un utilisateur, le procédé comprenant les étapes suivantes :
- mesurer la pression de l'espace confiné d'air ;
- établir à partir de la mesure de pression des caractéristiques respiratoires de l'utilisateur ;
- comparer la caractéristique respiratoire à au moins une valeur cible;
- préconiser un ajustement du mode respiratoire de l'utilisateur,
le masque étant tel qu'introduit ci-dessus.

Selon l'invention, les caractéristiques respiratoires sont la pression maximale dans l'espace confiné lors d'une expiration de valeur cible comprise entre 8 et 12 cmH2O, la durée d'une expiration de valeur cible comprise entre 3,5 et 4,5 secondes, et la durée d'une inspiration de valeur cible comprise entre 1,5 et 2,5 secondes.

Selon d'autres caractéristiques avantageuses et non limitatives de l'invention, prises seules ou en combinaison :
- le procédé comprend l'allumage d'au moins un témoin lumineux ou l'émission d'un son ou d'un message vocal, ou l'affichage d'un texte ou d'un graphique sur un écran.
- le procédé comprend également l'enregistrement de la pression mesurée et/ou de la caractéristique respiratoire.

### BREVE DESCRIPTION DES DESSINS

L'invention sera mieux comprise à la lumière de la description qui suit des modes de réalisation particuliers et non limitatifs de l'invention en référence aux figures ci-jointes parmi lesquelles :
- la figure 1 représente un masque de respiration d'un dispositif d'acclimatation à l'altitude conforme à l'invention ;
- la figure 2 représente une vue en perspective du masque de respiration de la figure 1 ;
- les figures 3 à 5 représentent des exemples particuliers du dispositif selon l'invention.

### DESCRIPTION DETAILLEE DE L'INVENTION

D'une manière très générale, le dispositif d'acclimatation à l'altitude 1 conforme à l'invention comprend un masque de respiration 2 à placer sur le visage d'un utilisateur, et un organe de traitement 3 configuré pour établir une caractéristique respiratoire de cet utilisateur, en vue de lui préconiser un ajustement de son mode respiratoire.

Dans le cadre de la présente demande, on désignera par caractéristique respiratoire toute information mesurée ou calculée se rapportant à l'air inspiré/expiré par l'utilisateur. Il peut s'agir, par exemple, de la quantité d'air inspiré ou expiré, de la pression de cet air dans le masque 2, du temps inspiratoire et/ou expiratoire, du rythme respiratoire (c'est-à-dire du nombre d'inspiration/expiration par unité de temps). La combinaison des caractéristiques respiratoires d'un utilisateur forme, selon la présente demande, son mode respiratoire. Un ajustement du mode respiratoire peut donc correspondre à la modification d'un ou d'une pluralité de caractéristiques.

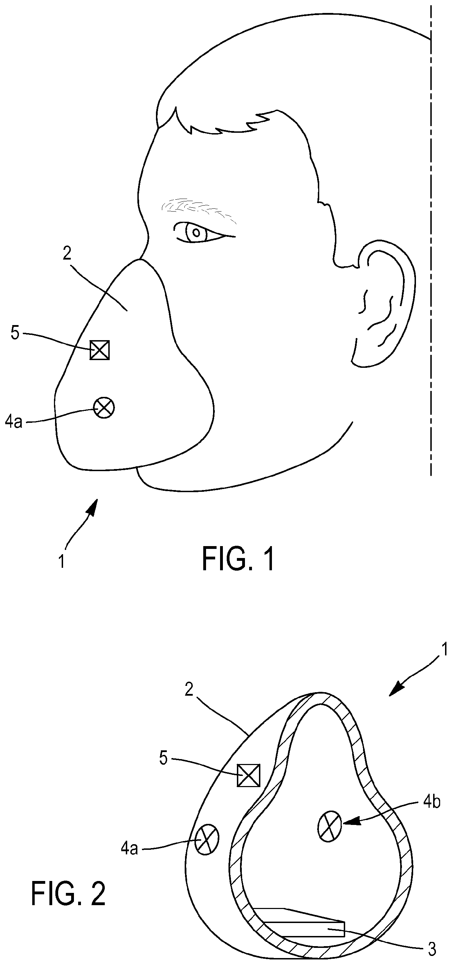

La figure 1 représente un masque de respiration 2 d'un dispositif d'acclimatation à l'altitude placé sur le visage d'un utilisateur. La figure 2 représente une vue en perspective du masque 2. Comme cela est visible sur la figure 1, le masque 2 est configuré pour englober la bouche et le nez de l'utilisateur. Et comme cela sera détaillé plus en détail par la suite, il présente des orifices pour laisser entrer l'air inspiré et sortir l'air expiré.

Le masque 2 est fixé sur le visage par des moyens adéquats, tels que des sangles (non représentées sur les figures), avantageusement ajustables à la morphologie de l'utilisateur. Une fois le masque 2 positionné, son contour 2a est en appui sur une zone d'appui périphérique du visage de l'utilisateur. La force d'appui est préférentiellement choisie pour que le port du masque 2 reste confortable à son utilisateur tout en assurant l'étanchéité suffisante du masque au niveau de la zone d'appui. On définit ainsi à l'intérieur du masque, un espace confiné délimité par la surface intérieure du masque 2 et le visage de l'utilisateur.

Par étanchéité suffisante, on signifie que l'air expiré ou inspiré n'est pas susceptible de passer entre le contour 2a du masque et la zone périphérique d'appui en conditions normales d'utilisation, même lorsque l'utilisateur respire dans des conditions de surpression expiratoire. Ainsi, et à titre d'exemple, l'étanchéité entre la zone périphérique d'appui et le contour 2a du masque 2 peut être maintenue pour une différence de pression entre l'espace confiné et l'environnement extérieur allant jusqu'à 15 cmH2O (1470 Pa), voire même 20 cmH20 (1960 Pa) .

Avantageusement, pour les raisons évoquées de confort et d'étanchéité, le contour 2a du masque peut être muni d'un joint d'appui (non représenté sur la figure 2).

Comme cela a été mentionné brièvement, le masque de respiration 2 est muni d'un premier et d'un second orifice. Selon l'invention, le premier orifice est muni d'une valve d'inspiration 4a pour laisser entrer de l'air environnant à l'intérieur de l'espace confiné lors d'une inspiration. Le second orifice est muni quant à lui d'une valve d'expiration 4b pour laisser sortir de l'air avec une résistance ou valeur de pression seuil déterminée lors d'une expiration, de sorte à créer une surpression d'air dans l'espace confiné du masque 2.

Ainsi, l'inspiration de l'utilisateur crée une légère sous-pression à l'intérieur du masque 2 conduisant à l'ouverture de la valve d'inspiration 4a et à la fermeture de la valve d'expiration 4b, permettant ainsi à l'air environnant d'entrer dans l'espace confiné librement, avant d'être inspiré dans les poumons de l'utilisateur.

Similairement, l'expiration de l'utilisateur crée une surpression dans l'espace confiné du masque 2 conduisant à la fermeture de la valve d'inspiration 4a. La valve d'expiration 4b quant à elle ne s'ouvre que lorsque la surpression de l'espace confiné dépasse la valeur de pression seuil déterminée.

Lors de l'utilisation conforme du masque 2, on forme de la sorte pendant la phase d'expiration une surpression dans l'espace confiné qui favorise durablement l'ouverture des alvéoles des poumons de l'utilisateur, et permet d'augmenter l'apport en oxygène à l'organisme lors des phases d'inspiration suivantes.

La valve d'expiration 4b peut être configurée pour que la résistance déterminée corresponde à une surpression de l'espace confiné d'environ 10 cmH2O (980 Pa). D'une manière plus générale, la résistance déterminée peut être comprise entre 5 (490 Pa) et 20 cmH20 (1960 Pa).

De manière avantageuse, la résistance déterminée de la valve d'expiration 4b est ajustable par l'utilisateur. À titre d'exemple, on peut prévoir :
- une valve d'expiration 4b présentant une première configuration pour laquelle la résistance déterminée correspond à une première surpression de l'espace confiné, par exemple 5 cmH20 (490 Pa); et
- une seconde configuration pour laquelle la résistance déterminée correspond à une seconde surpression de l'espace confiné supérieure à la première, par exemple 10 cmH20 (980 Pa).

On peut de la sorte proposer un dispositif respiratoire 1 unique pouvant être utilisé par des utilisateurs présentant des morphologies très différentes, et obtenir une acclimatation à l'altitude satisfaisante dans tous les cas.

Les valves 4a, 4b d'inspiration et d'expiration sont des dispositifs courants, bien connus en soi, et sont typiquement formés de clapets mécaniques réagissant à la différence de pression existant de part et d'autre du clapet, pour ouvrir ou fermer un passage.

D'autres formes de mise en œuvre des valves 4a, 4b sont bien entendu envisageables dans le cadre de la présente invention pour assurer les fonctions qui viennent d'être décrites.

Selon l'invention, le masque 2 est également pourvu d'un capteur 5 pour fournir une information de pression de l'espace confiné. Il peut être placé et fixé sur la surface intérieure du masque pour avoir un accès direct à l'espace confiné et en déterminer la pression. Il peut être également placé dans un logement formé dans une paroi du masque, le logement débouchant dans l'espace confiné afin d'en permettre la mesure ou en tout autre endroit facilitant cette mesure.

Avantageusement, le capteur 5 fournit l'information de pression sous forme électrique ou numérique, afin de permettre son traitement par l'organe de traitement 3. Cette information peut être fournie de manière continue ou avec une fréquence suffisante vis-à-vis des phénomènes mesurés pour permettre d'en suivre l'évolution. Ainsi, la fréquence de mise à disposition des informations peut être comprise entre 0,1 Hz et 10 Hz.

Le dispositif d'acclimatation à l'altitude 1 selon l'invention comprend également un organe de traitement 3 de l'information de pression de l'espace confiné fourni par le capteur 5. À cet effet, le capteur 5 et l'organe 3 sont connectés l'un à l'autre par l'intermédiaire d'une liaison. Cette liaison peut correspondre une simple liaison électrique, un bus de données dans le cas où les informations sont numériques, voire même à une liaison sans fil, par exemple selon la norme Bluetooth™. Dans le cas d'une telle liaison, le capteur 5 est muni au moins de moyens d'émission, et l'organe 3 de moyens de réception.

L'organe de traitement 3 peut être fixé sur le masque 2, par exemple à l'intérieur de celui-ci comme cela est représenté sur la figure 2, mais l'invention n'est nullement limitée à cette configuration. Ainsi, l'organe de traitement 3 peut également être fixé à l'extérieur du masque. Ces deux configurations présentent l'avantage de constituer un dispositif 1 compact. Dans ce cas, le masque est pourvu d'une source d'énergie, telle qu'une batterie ou une pile, permettant d'alimenter les éléments actifs tels que l'organe de traitement 3, et ses éventuels périphériques.

Alternativement, l'organe 3 peut être compris dans un dispositif distinct 8 du masque 2 auquel il peut être relié par un câble de raccordement ou par une liaison sans fil. Le dispositif distinct 8 peut par exemple être une oreillette ou un autre dispositif pouvant être tenu à la main par l'utilisateur ou maintenu à son poignet.

Les fonctions réalisées par l'organe de traitement 3 peuvent être implémentées de diverses formes. Ainsi l'organe peut être constitué de composants logique et électronique intégrés ou non, ou constitué d'une structure à microcontrôleur exécutant un programme enregistré dans une mémoire, ou d'une structure matérielle programmable du type FPGA.

Dans le cas où l'organe 3 est compris ou correspond à un dispositif distinct 8 du masque 2, il peut être avantageux d'implémenter ses fonctions sous la forme d'un programme s'exécutant sur un matériel préexistant (téléphone, montre, tablette...) .

Quel que soit le mode de réalisation de cet organe 3, les informations de pression de l'espace confiné d'air dans le masque 2 y sont traitées en vue de préconiser à l'utilisateur un ajustement de son mode respiratoire. Cet ajustement vise à améliorer ou accélérer l'acclimatation à l'altitude. Ceci peut être mesuré par exemple par l'évolution de la saturation artérielle en oxygène. Une utilisation conforme du dispositif 1 selon l'invention pouvant conduire, en moyenne, et à une altitude de 4500 m, à augmenter cette valeur de 6 % ou plus, ce qui est significatif.

L'organe de traitement a donc pour fonction principale d'assurer une utilisation conforme du dispositif. Pour ce faire, cet organe établi, à partir de l'information de pression, des caractéristiques respiratoires de l'utilisateur qu'il compare à des valeurs cibles pour établir une préconisation d'ajustement de son mode respiratoire. Plus précisément, l'organe de traitement 3 met en œuvre un procédé qui comporte les étapes suivantes :
- la réception de la mesure de la pression d'un espace confiné d'air défini par le masque placé sur le visage de l'utilisateur ;
- l'établissement, à partir de la mesure de pression, de caractéristiques du mode respiratoire de l'utilisateur ;
- la comparaison respective des caractéristiques respiratoires à une valeur cible en vue de la préconisation à l'utilisateur d'un ajustement de son mode respiratoire.

Dans le cadre de la présente demande, le terme « préconisation » et « préconiser » correspondent à l'emploi de tous moyens techniques permettant de communiquer à l'utilisateur des instructions ou des informations relatives à son mode respiratoire, notamment pour l'ajuster et employer ainsi de manière conforme le dispositif d'acclimatation 1. D'une manière générale, ces préconisations d'ajustements du mode respiratoire sont communiquées à l'utilisateur à l'aide d'un organe de préconisation 6, relié à l'organe de traitement 3.

Une première caractéristique respiratoire établie par l'organe de traitement 3 correspond à la pression maximale dans l'espace confinée atteinte lors d'une expiration. Les études menées par les inventeurs de la présente demande ont montré que cette caractéristique devait préférablement être comprise entre 8 et 12 cmH2O (entre 784 Pa et 1176 Pa).

Si au cours de son traitement, l'organe 3 identifie par comparaison que la pression maximale est inférieure à 8 cmH2O, il sera préconisé à l'utilisateur d'expirer avec plus de force. Si au contraire, l'organe 3 identifie par comparaison que la pression maximale est supérieure à 12 cmH2O, il sera préconisé à l'utilisateur d'expirer avec moins de force.

Finalement, si l'organe 3 identifie à l'issue de ces comparaisons que la pression maximale et bien comprise entre 8 et 12 cmH2O, il n'est alors pas nécessaire de communiquer de préconisation particulière à l'utilisateur, si ce n'est de confirmer que cette caractéristique respiratoire est bien conforme.

Une seconde caractéristique respiratoire est la durée d'une expiration et la valeur cible est comprise entre 3,5 secondes et 4,5 secondes. La durée d'une expiration peut être établie par l'organe 3 en déterminant la durée pendant laquelle l'information fournie par le capteur 5 correspond à une pression supérieure à la pression environnante. Les préconisations peuvent alors correspondre à un allongement ou à une réduction, selon le cas, de la durée de l'expiration.

Une troisième caractéristique respiratoire, découlant de la seconde, est la durée d'une inspiration. La valeur cible correspondante est comprise entre 1,5 et 2,5 secondes. Similairement au cas précédent, la durée de l'inspiration peut être établie par l'organe 3 en déterminant la durée pour laquelle l'information fournie par le capteur 5 correspond à une pression dans l'espace confiné inférieure à la pression environnante. Les préconisations peuvent alors correspondre à une réduction ou un allongement, selon le cas, de la durée d'inspiration.

On notera qu'une respiration naturelle, sans application pression expiratoire, conduit à une durée d'expiration de l'ordre de 2,5 secondes et une durée d'inspiration de l'ordre de 2 seconde. Dans ces conditions de respiration naturelle, l'oxygénation artérielle d'un usager à 4300m d'altitude estimée par une mesure de saturation pulsée en oxygène (et communément représenté par le terme SpO2) est typiquement comprise entre 75% et 80%. On rappelle que la mesure de SpO2 peut être facilement obtenue à l'aide d'un oxymètre de pouls, et que cette mesure établie en % représente le pourcentage d'hémoglobine oxygénée par rapport a la quantité totale d'hémoglobine dans le sang.

Les inventeurs de la présente demande ont établi que lorsque la pression expiratoire était bien contrôlée entre 8 et 12 cm H2O, le temps expiratoire bien contrôlée entre 3,5 et 4,5 secondes, et le temps d'inspiration bien compris entre 1,5 et 2,5 secondes, la mesure de SpO2 révélait un taux supérieur à 80%, et proche de 85%, ce qui montre bien l'effet particulièrement notable de ce mode respiratoire sur l'oxygénation du sang, et sur l'acclimatation d'une personne à l'altitude.

Bien entendu, les traitements réalisés par l'organe 3 ne sont pas limités aux exemples qui ont été donnés ci-dessus. Notamment, le masque 2 peut comporter, ou l'utilisateur être équipé, d'autres capteurs pouvant également contribuer à l'établissement par l'organe 3 de préconisations d'ajustement du mode respiratoire. L'organe 3 peut être également configuré pour enregistrer les données reçues et/ou traitées en vue d'une exploitation ultérieure.

Ainsi, et à titre d'exemple, l'utilisateur peut être équipé d'un capteur de rythme cardiaque ou encore d'un oxymètre qui, comme dans le cas du capteur 5, peuvent être reliés à l'organe de traitement 3 à l'aide d'une liaison filaire ou sans fil. La disponibilité d'un oxymètre (qui peut être placé sur l'oreille ou le doigt de l'utilisateur) présente l'avantage de pouvoir accéder à une mesure objective de l'acclimatation à l'altitude de l'utilisateur. Cette mesure peut lui être communiquée pour lui confirmer ou non l'utilisation conforme du dispositif 1 et l'informer de son état physiologique d'acclimatation à l'altitude.

De manière avantageuse, l'organe de préconisation 6 comprend des moyens visuels de communication des instructions à l'utilisateur. Il peut s'agir d'une pluralité de témoins lumineux, éventuellement de couleurs différentes, placées sur le masque et visibles de l'utilisateur. Alternativement ou en complément, il peut s'agir d'un écran tenu à la main ou maintenu au poignet sur lequel peut être affichée l'instruction sous forme de texte ou de graphique.

Alternativement, ou en complément, l'organe de préconisation 6 comprend des moyens sonores de communication. Il peut s'agir d'un haut-parleur placé sur le masque 2 ou d'une oreillette placée sur l'utilisateur lui-même, l'un ou l'autre étant en communication avec l'organe 3. Ces communications peuvent correspondre à une pluralité de sons, chacun de ces sons étant associé à une préconisation particulière préétablie, mais avantageusement ces communications sont des messages préenregistrés.

### Exemple 1

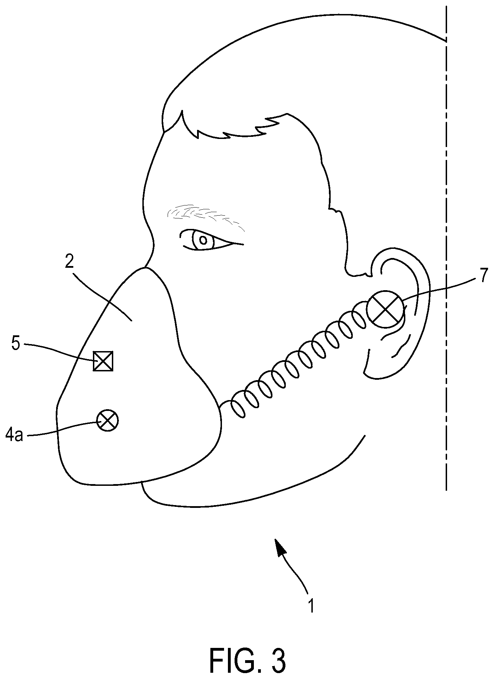

Sur la figure 3, un masque 2 placé sur le visage d'un utilisateur est muni de valves 4a, 4b d'inspiration et d'expiration et d'un capteur 5 de pression (seule la valve 4a est visible sur la figure 3). L'organe 3 de traitement est quant à lui placé à l'intérieur du masque 2 (et donc non visible sur cette figure), dans sa partie inférieure. Cet organe est relié au capteur 5 par une liaison électrique simple. L'organe 3 est également relié à une oreillette 7 (l'organe de préconisation) placée à l'oreille de l'utilisateur afin de lui communiquer sous la forme de messages préenregistrés les préconisations d'ajustement de son mode respiratoire. L'organe 3 est, dans le cas de cet exemple 1, constitué d'un microcontrôleur comprenant des ports d'entrée-sortie pour établir les liaisons avec le capteur 5 et l'oreillette 7. Il comprend également une mémoire non volatile dans lequel réside le programme de traitement, les messages préenregistrés, et dans lesquelles peuvent être enregistrées les informations de pression et les caractéristiques respiratoires établies au cours d'une sortie pour leur exploitation ultérieure.

### Exemple 2

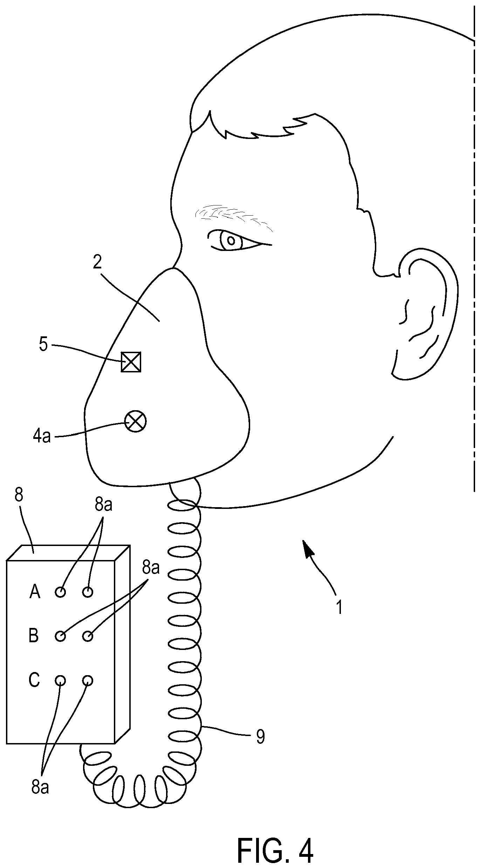

La figure 4 présente un second exemple d'un dispositif 1 conforme à l'invention. Le masque 2 de l'exemple 2 est en tout point similaire à celui de l'exemple 1. Toutefois, dans cet exemple 2, les préconisations d'ajustement du mode respiratoire sont communiquées à l'utilisateur à l'aide d'un dispositif 8 distinct du masque 2 et relié à l'organe de traitement 3 par l'intermédiaire d'une liaison filaire 9. Le dispositif 8 peut être tenu à la main ou maintenu au poignet de l'utilisateur par exemple. Il comprend une pluralité de témoins lumineux 8a, chacun de ces témoins indiquant une préconisation prédéfinie ou se référent à une caractéristique prédéfinie, qui peut être inscrite à proximité du témoin lumineux. Dans une variante de cet exemple, la liaison filaire 9 peut être remplacée par une liaison sans fil.

### Exemple 3

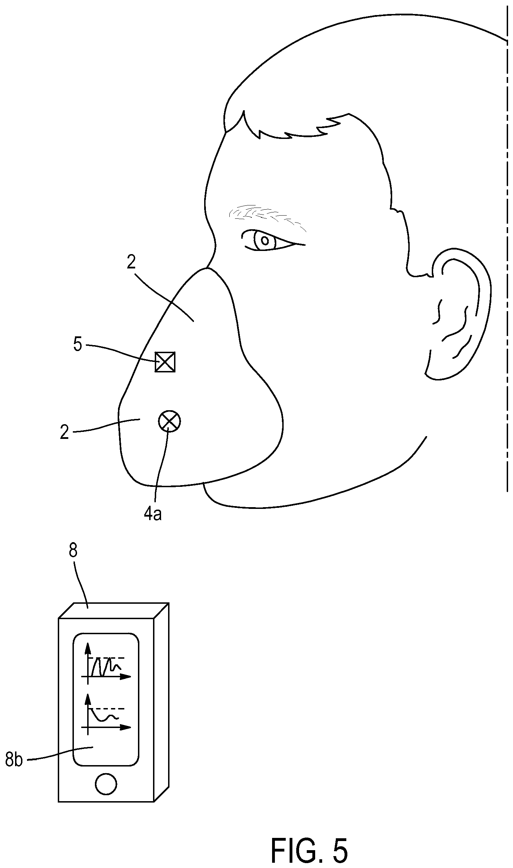

La figure 5 est présente un autre exemple d'un dispositif 1 conforme à l'invention. Dans cet exemple, le masque 2 comporte les valves 4a, 4b (mais seul la valve 4a est visible sur cette figure) et le capteur 5 similaires au masque des exemples précédents. Le capteur 5 est muni de moyens de communication des informations à un dispositif distinct 8 de calcul. Avantageusement, le dispositif du calcul 8 est un téléphone, une tablette, ou encore une montre connectée. Une application s'exécutant sur ces moyens de calcul traite les informations de pression reçue et présente les préconisations d'ajustement du mode respiratoire sur un écran d'affichage 8b, par exemple sous la forme d'un graphique ou de texte.

Bien entendu l'invention n'est pas limitée aux modes de mise en œuvre décrits et on peut y apporter des variantes de réalisation sans sortir du cadre de l'invention tel que défini par les revendications.

## Revendications

1. Dispositif d'acclimatation à l'altitude (1) comprenant un masque de respiration (2) définissant un espace confiné d'air lorsqu'il est placé sur le visage d'un utilisateur, le masque (2) comportant :
- Une valve d'inspiration (4a) pour laisser entrer de l'air environnant à l'intérieur de l'espace confiné lors d'une inspiration ;
- Une valve d'expiration (4b) pour laisser sortir de l'air avec une résistance déterminée, de sorte à créer une surpression d'air dans l'espace confiné, lors d'une expiration ;
- Un capteur (5) pour établir une information de pression de l'espace confiné d'air ;
le dispositif d'acclimatation (1) comportant de plus un organe de traitement (3) de l'information de pression configuré pour établir des caractéristiques respiratoires de l'utilisateur et les comparer, respectivement, à au moins une valeur cible, en vue de lui préconiser un ajustement de son mode respiratoire, le dispositif étant **caractérisé en ce que** les caractéristiques respiratoires comprennent la pression maximale dans l'espace confiné lors d'une expiration de valeur cible comprise entre 8 et 12 cmH2O, la durée d'une expiration de valeur cible comprise entre 3,5 et 4,5 secondes, et la durée d'une inspiration de valeur cible comprise entre 1,5 et 2,5 secondes.

2. Dispositif d'acclimatation (1) selon la revendication précédente comprenant également un oxymètre en liaison avec l'organe de traitement (3).

3. Dispositif d'acclimatation (1) selon l'une des revendications précédentes comprenant également un organe de préconisation (6) en liaison avec le dispositif de traitement (3).

4. Dispositif d'acclimatation (1) selon l'une des revendications précédentes dans lequel l'organe de traitement (3) est disposé dans ou sur le masque (2).

5. Dispositif d'acclimatation (1) selon l'une des revendications 1 à 4 dans lequel l'organe de traitement (3) est disposé ou est constitué par un dispositif distinct du masque (2).

6. Procédé de fonctionnement d'un dispositif d'acclimatation à l'altitude (1) comprenant un masque de respiration (2) définissant un espace confiné d'air lorsqu'il est placé sur le visage d'un utilisateur, le masque (2) comportant :
- Une valve d'inspiration (4a) pour laisser entrer de l'air environnant à l'intérieur de l'espace confiné lors d'une inspiration ;
- Une valve d'expiration (4b) pour laisser sortir de l'air avec une résistance déterminée, de sorte à créer une surpression d'air dans l'espace confiné, lors d'une expiration ;
- Un capteur (5) pour établir une information de pression de l'espace confiné d'air ; et
- un organe de traitement (3) de l'information de pression,
le procédé comprenant les étapes suivantes :
- mesurer la pression de l'espace confiné d'air ;
- établir à partir de la mesure de pression des caractéristiques respiratoires de l'utilisateur ;
- comparer chaque caractéristique respiratoire à au moins une valeur cible ;
- préconiser un ajustement du mode respiratoire de l'utilisateur ;
le procédé étant **caractérisé en ce que** les caractéristiques respiratoires sont la pression maximale dans l'espace confiné lors d'une expiration de valeur cible comprise entre 8 et 12 cmH2O, la durée d'une expiration de valeur cible comprise entre 3,5 et 4,5 secondes, et la durée d'une inspiration de valeur cible comprise entre 1,5 et 2,5 secondes.

7. Procédé de fonctionnement d'un dispositif d'acclimatation (1) selon la revendication précédente dans lequel l'étape de préconisation comprend l'allumage d'au moins un témoin lumineux ou l'émission d'un son ou d'un message vocal, ou l'affichage d'un texte ou d'un graphique sur un écran.

8. Procédé de fonctionnement d'un dispositif d'acclimatation (1) selon l'une des deux revendications précédentes dans lequel le procédé comprend également l'enregistrement de la pression mesurée et/ou de la caractéristique respiratoire.

## Patentansprüche

1. Vorrichtung zum Akklimatisieren an die Höhenlage (1), umfassend eine Atemmaske (2), die einen umschlossenen Luftraum definiert, wenn sie auf das Gesicht eines Benutzers aufgesetzt wird, wobei die Maske (2) umfasst:
- ein Einatemventil (4a), um bei einem Einatmen Umgebungsluft in das Innere des umschlossenen Raums einströmen zu lassen;
- ein Ausatemventil (4b), um bei einem Ausatmen Luft mit einem bestimmten Widerstand so ausströmen zu lassen, dass in dem umschlossenen Raum ein Luftüberdruck erzeugt wird;
- einen Sensor (5), um eine Druckinformation von dem umschlossenen Luftraum zu erfassen;
wobei die Akklimatisierungsvorrichtung (1) darüber hinaus eine Einrichtung zum Verarbeiten (3) der Druckinformation umfasst, die dafür konfiguriert ist, Atemmerkmale des Benutzers zu erfassen und dieselben jeweils mit mindestens einem Zielwert zu vergleichen, um ihm eine Anpassung seines Atemmodus zu empfehlen, wobei die Vorrichtung **dadurch gekennzeichnet ist, dass** die Atemmerkmale den maximalen Druck im umschlossenen Raum bei einem Ausatmen mit Zielwert im Bereich zwischen 8 und 12 cmH2O, die Dauer eines Ausatmens mit Zielwert im Bereich zwischen 3,5 und 4,5 Sekunden, und die Dauer eines Einatmens mit Zielwert im Bereich zwischen 1,5 und 2,5 Sekunden umfassen.

2. Akklimatisierungsvorrichtung (1) nach dem vorstehenden Anspruch, die ebenfalls einen Sauerstoffmesser in Verbindung mit der Verarbeitungseinrichtung (3) umfasst.

3. Akklimatisierungsvorrichtung (1) nach einem der vorstehenden Ansprüche, die ebenfalls eine Empfehlungseinrichtung (6) in Verbindung mit der Verarbeitungsvorrichtung (3) umfasst.

4. Akklimatisierungsvorrichtung (1) nach einem der vorstehenden Ansprüche, wobei die Verarbeitungseinrichtung (3) in oder an der Maske (2) angeordnet ist.

5. Akklimatisierungsvorrichtung (1) nach einem der Ansprüche 1 bis 4, wobei die Verarbeitungseinrichtung (3) angeordnet ist an oder gebildet wird von einer Vorrichtung, die von der Maske (2) getrennt ist.

6. Verfahren zum Betrieb einer Vorrichtung zum Akklimatisieren an die Höhenlage (1), welche eine Atemmaske (2) umfasst, die einen umschlossenen Luftraum definiert, wenn sie auf das Gesicht eines Benutzers aufgesetzt wird, wobei die Maske (2) umfasst:
- ein Einatemventil (4a), um bei einem Einatmen Umgebungsluft in das Innere des umschlossenen Raums einströmen zu lassen;
- ein Ausatemventil (4b), um bei einem Ausatmen Luft mit einem bestimmten Widerstand so ausströmen zu lassen, dass in dem umschlossenen Raum ein Luftüberdruck erzeugt wird;
- einen Sensor (5), um eine Druckinformation von dem umschlossenen Luftraum zu erfassen; und
- eine Einrichtung zum Verarbeiten (3) der Druckinformation,
wobei das Verfahren die folgenden Schritte umfasst:
- Messen des Drucks des umschlossenen Luftraums;
- Erfassen, auf Grundlage der Druckmessung, von Atemmerkmalen des Benutzers;
- Vergleichen jedes Atemmerkmals mit mindestens einem Zielwert;
- Empfehlen einer Anpassung des Atemmodus für den Benutzer;
wobei das Verfahren **dadurch gekennzeichnet ist, dass** die Atemmerkmale der maximale Druck im umschlossenen Raum bei einem Ausatmen mit Zielwert im Bereich zwischen 8 und 12 cmH2O, die Dauer eines Ausatmens mit Zielwert im Bereich zwischen 3,5 und 4,5 Sekunden, und die Dauer eines Einatmens mit Zielwert im Bereich zwischen 1,5 und 2,5 Sekunden sind.

7. Betriebsverfahren einer Akklimatisierungsvorrichtung (1) nach dem vorstehenden Anspruch, wobei der Schritt des Empfehlens das Aufleuchten von mindesten einem Kontrolllämpchen oder das Ausgeben eines Tons oder einer Sprachnachricht, oder das Anzeigen eines Textes oder einer Grafik auf einem Bildschirm umfasst.

8. Betriebsverfahren einer Akklimatisierungsvorrichtung (1) nach einem der zwei vorstehenden Ansprüche, wobei das Verfahren ebenfalls das Aufzeichnen des gemessenen Drucks und/oder des Atemmerkmals umfasst.

## Claims

1. Device for acclimatising at altitude (1) comprising a breathing mask (2) defining a confined air space when it is placed on the face of a user, the mask (2) comprising:
- An inhalation valve (4a) to let the surrounding air enter inside the confined space during an inhalation;
- An exhalation valve (4b) to let air exit with a determined resistance, so as to create an air overpressure in the confined space, during an exhalation;
- A sensor (5) to establish pressure information of the confined air space;
the acclimatising device (1) comprising, in addition, a member for processing (3) pressure information configured to establish breathing characteristics of the user and compare them, respectively, to at least one target value, in view of recommending them an adjustment of their breathing mode, the device being **characterised in that** the breathing characteristics comprise the maximum pressure in the confined space during an exhalation of target value of between 8 and 12cmH₂O, the duration of an exhalation of target value of between 3.5 and 4.5 seconds, and the duration of an inhalation of target value of between 1.5 and 2.5 seconds.

2. Acclimatising device (1) according to the preceding claim, also an oximeter connected to the processing member (3).

3. Acclimatising device (1) according to one of the preceding claims, also comprising a recommendation member (6) connected to the processing device (3).

4. Acclimatising device (1) according to one of the preceding claims, wherein the processing member (3) is arranged in or on the mask (2).

5. Acclimatising device (1) according to one of claims 1 to 4, wherein the processing member (3) is arranged or constituted by a device separate from the mask (2).

6. Method for operating a device for acclimatising at altitude (1) comprising a breathing mask (2) defining a confined air space when it is placed on the face of a user, the mask (2) comprising:
- An inhalation valve (4a) to let the surrounding air enter inside the confined space during an inhalation;
- An exhalation valve (4b) to let air exit with a determined resistance, so as to create an air overpressure in the confined space, during an exhalation;
- A sensor (5) to establish pressure information of the confined air space; and
- A member for processing (3) the pressure information, the method comprising the following steps:
- measuring the pressure of confined air space;
- establishing from the pressure measurement of the breathing characteristics of the user;
- comparing each breathing characteristic with at least one target value;
- recommending an adjustment of the breathing mode of the user;
the method being **characterised in that** the breathing characteristics are the maximum pressure in the confined space during an exhalation of target value of between 8 and 12cmH₂O, the duration of an exhalation of target value of between 3.5 and 4.5 seconds, and the duration of an inhalation of target value of between 1.5 and 2.5 seconds.

7. Method for operating an acclimatising device (1) according to the preceding claim, wherein the recommendation step comprises the illumination of at least one warning light or the emission of a sound or of a vocal message, or the displaying of a text or a graphic on a screen.

8. Method for operating an acclimatising device (1) according to one of the two preceding claims, wherein the method also comprises the recording of the pressure measured and/or the breathing characteristic.
